# EUROPÄISCHE PATENTANMELDUNG

(11) **EP 4 714 475 A1**
(43) Veröffentlichungstag der Anmeldung: **25.03.2026**
(21) Anmeldenummer: 24201035.3
(22) Anmeldetag: 18.09.2024
(51) Int. Cl.: A61M 1/06

(54) **MILCHPUMPE**

(71) Anmelder: MAM Baby AG, 8832 Wollerau (CH)
(72) Erfinder: PINTER, Roland, 7022 Schattendorf (AT)
(74) Vertreter: SONN Patentanwälte GmbH & Co KG

(57) **Zusammenfassung**

Milchpumpe (1) aufweisend:
- einen Milchbehälter (2) mit einem Hohlraum (3) zur Aufnahme von abgepumpter Milch und mit einer umlaufenden Mündungswandung (4), der eine Öffnung (5) zum Hohlraum (3) bildet;
- eine Brusthaube (30), die einen Brusttrichter (32) und einen Nippeltunnel (31) aufweist;
- ein Verbindungselement (6), das die Öffnung (5) zum Hohlraum (3) des Milchbehälters (2) mit dem Nippeltunnel verbindet;
- ein einstückiges Ventil- und Dichtungselement (7), das ein Rückschlagventil (8), einen sich vom Rückschlagventil (8) erstreckenden umlaufenden Verbindungssteg (9) und ein an den Verbindungssteg (9) anschließendes umlaufendes Dichtelement (10) aufweist,
wobei das Dichtelement (10) vollumfänglich an der Mündungswandung (4) anliegt.

## Beschreibung

Die Erfindung betrifft eine Milchpumpe aufweisend:
- einen Milchbehälter mit einem Hohlraum zur Aufnahme von abgepumpter Milch und mit einer umlaufenden Mündungswandung, der eine Öffnung zum Hohlraum bildet;
- eine Brusthaube, die einen Nippeltunnel und vorzugsweise einen Brusttrichter aufweist; und
- ein Verbindungselement, das die Öffnung zum Hohlraum des Milchbehälters mit dem Nippeltunnel verbindet.

Eine solche Brustpumpe ist beispielsweise in der EP 4066870 A2 gezeigt. Der Brusttrichter wird auf die Brust aufgesetzt. Durch die Erzeugung eines Unterdrucks wird Milch aus der Brust abgepumpt, die über den Verbindungselement mit einer Öffnung in den Hohlraum des Milchbehälters fließt. Der Milchbehälter weist eine große Öffnung auf, die mit einer Kappe mit integrierter Tülle verschlossen wird. Ein flexibles Gummiventil ist an der Tülle angebracht und umfasst ein Entenschnabelventil, durch das Milch nur in die Flasche fließen, aber nicht auslaufen kann. Die Kappe kann in die Milchsammelflasche eingeschraubt werden. Nachteiligerweise kann jedoch Milch zwischen der Kappe und dem Milchsammelbehälter austreten. Darüber hinaus ist die Montage kompliziert, da nicht nur eine Kappe, sondern auch ein Entenschnabelventil ausgerichtet und montiert werden müssen. Da die Kappe und das Entenschnabelventil getrennt ausgeführt sind, könnte auch Milch zwischen diesen beiden auslaufen. Auch die Reinigung ist durch den komplexen Aufbau der beiden Teile (Kappe und Element mit Entenschnabelventil) schwierig.

US 2018/0093024 A1 zeigt eine Milchpumpe mit einem Brustflansch, einem Adapter, einer Milchsammelvorrichtung und einem Ventil zwischen Adapter und Milchsammelvorrichtung. Das Ventil dient dazu, ein Vakuum im Adapter aufrecht zu halten. Das Ventil weist Halterippen auf, die um den Umfang des Ventils angeordnet sind. Nachteiligerweise sind die Rippen durch einen Entlüftungskanal unterbrochen, der zu einer Entlüftung führt. Durch den Entlüftungskanal kann Umgebungsluft in die Milchsammelvorrichtung strömen. Nachteiligerweise kann dadurch jedoch auch Milch (bspw. falls die Milchpumpe längere Zeit schräg liegt) aus dem Milchbehälter über den Entlüftungskanal zum Gewinde und nach außen austreten. Selbst bei einem kürzeren Schräghalten kann eine gewisse Menge Milch zumindest bis zum Gewinde rinnen. Aufgrund des Entlüftungskanals liegt der Ventilstopprand (Bezugszeichen 330, 425a, 525a) nicht vollumfänglich an der Milchsammelvorrichtung an.

WO 2017/157701 A1 zeigt eine weitere Milchpumpe. Diese hat einen Trichter zur Aufnahme der Brust, ein Gehäuse, einen Behälter zur Aufnahme der Muttermilch, einen Anschlussstutzen zum Anschluss an eine Vakuumquelle und ein Membranelement, das im Gehäuse zwischen dem Trichter und dem Behälter angeordnet ist. Die Dichtung weist einen Entlüftungskanal auf, über den Luft aus dem Behälter in die Umgebung austreten kann. Nachteiligerweise kann jedoch auch hier Milch zum Gewinde rinnen und aus dem Milchbehälter austreten.

Eine Möglichkeit zur Abdichtung des Milchbehälters zum Gewinde (an dem dieser mit dem Adapter zusammengeschraubt wird) hin, wäre es, einen umlaufenden Dichtring einzusetzen. Nachteiligerweise wäre damit bei der Montage ein zusätzliches Element vorzusehen, dessen genaue Platzierung schwierig wäre.

Eine Aufgabe der Erfindung liegt darin, einen oder mehrere der Nachteile des Stands der Technik zu beheben oder zu lindern. Es ist insbesondere eine Aufgabe der Erfindung, eine Milchpumpe vorzuschlagen, die einfach Zusammenzubauen ist, bei der das Auslaufen von Milch aus dem Milchbehälter gut verhindert wird und/oder die einfach zu reinigen ist.

Dies wird gelöst durch eine Milchpumpe wie eingangs angeführt, weiter aufweisend ein einstückiges Ventil- und Dichtungselement, das ein Rückschlagventil, einen sich vom Rückschlagventil erstreckenden umlaufenden Verbindungssteg und ein an den Verbindungssteg anschließendes umlaufendes Dichtelement aufweist, wobei das Dichtelement vollumfänglich an der Mündungswandung anliegt.

Dadurch das das Ventil- und Dichtungselement einstückig ausgeführt ist, und sowohl die Ventil-, als auch die Dichtungsfunktion übernimmt, ist der Zusammenbau der Milchpumpe vereinfacht. Weiters wird durch das umlaufende Dichtungselement, das vollumfänglich an der Mündungswandung anliegt, verhindert, dass abgepumpte Milch aus dem Milchbehälter austritt und bspw. in einen Gewindebereich des Milchbehälters und von dort nach außen fließen kann. Der Verbindungssteg dient der Abdichtung zwischen Dichtelement und Rückschlagventil. Durch die Einstückigkeit wird auch vermieden, dass weitere Stellen abgedichtet werden müssen.

Der Verbindungssteg schließt insbesondere vollumfänglich an das Rückschlagventil an. Das Dichtelement schließt insbesondere vollumfänglich an den Verbindungssteg an. Der Verbindungssteg ist insbesondere plattenförmig und/oder membranförmig ausgebildet. Das Dichtelement weist insbesondere einen (sich insbesondere weg von der Mündungswandung erstreckenden) Dichtflansch auf. Das Dichtelement liegt vorzugsweise entlang zumindest einer Linie vollumfänglich an der Mündungswandung an. Vorzugsweise liegt das Dichtelement an einer Stirnkante der Mündungswandung und/oder an einer inneren Mantelfläche der Mündungswandung vollumfänglich an. Die Linie, entlang derer das Dichtelement vollumfänglich an der Mündungswandung anliegt, kann auch (nur) teilweise an der Stirnkante und (nur) teilweise an der inneren Mantelfläche der Mündungswandung anliegen, sodass in Summe eine Abdichtung über den vollen Umfang bewirkt wird. Das Dichtelement liegt insbesondere derart vollumfänglich an der Mündungswandung an, dass eine vollständige Abdichtung zwischen Dichtelement und Milchbehälter erzielt wird. Vorzugsweise wird das Dichtelement vom Verbindungselement an der Mündungswandung angepresst, insbesondere vollumfänglich.

Die Mündungswandung kann insbesondere die Form der Mantelfläche eines Zylinders oder Kegels haben (also insbesondere hohlzylinderförmig oder hohlkegelförmig sein). Zur Befestigung des Milchbehälters an dem Verbindungselement können bspw. beide Elemente jeweils ein Gewinde aufweisen, die miteinander zusammenwirken. Insbesondere weist der Milchbehälter ein Außengewinde und das Verbindungselement ein Innengewinde auf. Es ist bspw. auch eine Verschnappung oder Bajonettverbindung zwischen Milchbehälter und Verbindungselement möglich.

Das Ventil- und Dichtungselement ist vorzugsweise in einem Stück hergestellt und/oder weist vorzugsweise keine separaten Teile auf, die zusammengefügt werden müssen. Das Ventil- und Dichtungselement besteht vorzugsweise aus einem durchgehenden Material und/oder ist vorzugsweise als ein durchgehendes Bauteil (bspw. durch eine durchgehende Fertigungsmethode wie z.B. Gießen, Schmieden oder Spritzgießen) hergestellt. Das Rückschlagventil, der Verbindungssteg und das Dichtelement weisen vorzugsweise das gleiche Material auf und bestehen bevorzugt aus dem gleichen Material. Vorzugsweise weist Ventil- und Dichtungselement Silikon auf, insbesondere besteht es aus Silikon. Vorzugsweise ist das Ventil- und Dichtungselement spritzgegossen, insbesondere in einem Stück. Das Ventil- und Dichtungselement weist eine Shore-Härte A von bevorzugt zwischen 30 und 70, besonders bevorzugt zwischen 40 und 60, auf. Das Rückschlagventil (Einwegeventil) ist vorzugsweise ein Schnabelventil (duckbill valve).

Das Verbindungselement weist vorzugsweise einen Verbindungsabschnitt auf, der zur Verbindung mit dem Nippeltunnel vorgesehen ist bzw. der den Nippeltunnel aufnimmt. Der Verbindungsabschnitt ist insbesondere allgemein zylinderförmig oder konisch ausgebildet. Die Brusthaube ist vorzugsweise mit dem Nippeltunnel in das Verbindungselement einsteckbar und wird in diesem durch Reibschluss gehalten. Vorzugsweise weist der Nippeltunnel im aus dem Verbindungsabschnitt entfernten Zustand einen Außenumfang auf, der gleich oder größer als ein Innenumfang des Verbindungsabschnitts ist. Durch die Übermaßigkeit des Nippeltunnels ist dieser vorteilhafterweise zum Verbindungselement dichtend. Vorzugsweise ist die Brusthaube vom Verbindungselement zerstörungsfrei trennbar. Der Verbindungsabschnitt hat vorzugsweise die Form eines allgemeinen Zylinders, insbesondere eines Kreiszylinders oder eines elliptischen Zylinders. Der Nippeltunnel hat vorzugsweise die Form eines allgemeinen Zylinders oder eines Kegelstumpfes (Konus). Der Verbindungsabschnitt des Verbindungselements ist vorzugsweise an den Nippeltunnel angepasst. Statt der allgemein zylindrischen Form kann auch eine kegelstumpfförmige oder pyramidenstumpfförmige Form für den Nippeltunnel und/oder den Verbindungsabschnitt vorgesehen sein. Weiters weist das Verbindungselement vorzugsweise ein Anschlusselement zur Verbindung mit dem Milchbehälter auf. Das Anschlusselement ist bevorzugt allgemein zylindrisch. Der Milchbehälter ist vorzugsweise mit dem Verbindungselement verbindbar, z.B. am Verbindungselement anschraubbar, ansteckbar oder über einen Bajonettverschluss verbindbar. Darunter, dass der Verbindungsabschnitt bzw. der Nippeltunnel allgemein zylinderförmig oder konisch ausgebildet sind, wird insbesondere verstanden, dass diese die Form der Mantelfläche eines allgemeinen Zylinders bzw. Konus (d.h. Kegelstumpfes) aufweisen.

Der Brusttrichter ist insbesondere zur (teilweisen) Aufnahme einer Brust eingerichtet. Der Milchbehälter ist vorzugsweise starr ausgebildet. Vorzugweise verläuft ein Milchflussweg vom Nippeltunnel bzw. von der Brusthaube über das Verbindungselement und über das Rückschlagventil zum Hohlraum des Milchbehälters. Beim Abpumpen wird die abgepumpte Milch entlang des Milchflusswegs geführt. Vorzugsweise ist eine Pumpe zur Erzeugung eines Unterdrucks in der Brusthaube bzw. im Nippeltunnel vorgesehen. Vorzugsweise bildet das Verbindungselement im Wesentlichen einen Milchflusskanal, der den Nippeltunnel mit dem Hohlraum verbindet. Vorzugsweise weist der Milchflusskanal eine Öffnung auf, über die ein Pumpenelement einen Unterdruck im Verbindungselement bzw. im Milchflusskanal erzeugen kann. Vorzugsweise ist eine Membran vorgesehen, über die das Pumpenelement über die Öffnung einen Unterdruck im Verbindungselement bzw. im Milchflusskanal erzeugen kann.

Bevorzugt weist der Nippeltunnel auf seinem äußeren Umfang eine Dichtgeometrie auf, bevorzugt zumindest eine oder zumindest zwei (insbesondere in Umfangsrichtung verlaufende) Dichtrippe(n) auf. Als Dichtgeometrie können bspw. (z.B. zusätzlich oder alternativ zu der/den Dichtrippen) insbesondere umlaufende Lamellen vorgesehen sein. Die zwei Dichtrippen sind dabei vorzugsweise vollumfänglich ausgebildet.

Die Brustpumpe ist vorzugsweise eine elektrische Brustpumpe.

Der Milchbehälter ist vorzugsweise starr ausgeführt, sodass das Volumen des Hohlraums beim Abpumpen im Wesentlichen unverändert bleibt. Somit ist der Milchbehälter vorzugsweise kein flexibler Beutel.

Es ist vorteilhaft, wenn das Dichtelement vollumfänglich an einer Stirnkante der Mündungswandung anliegt. Die Stirnkante der Mündungswandung liegt im Wesentlichen in derselben Ebene wie die Öffnung des Milchbehälters.

Es ist vorteilhaft, wenn das Dichtelement umlaufend um die Öffnung an dem Verbindungselement (insbesondere vollumfänglich) anliegt. Damit erfolgt eine Abdichtung zwischen Dichtelement und Verbindungselement. Somit kann Milch weder zwischen Milchbehälter und Dichtelement, noch zwischen Dichtelement und Verbindungselement nach außen austreten. Außerdem wird dadurch das Dichtelement, und damit das Ventil- und Dichtungselement, vom Verbindungselement und dem Milchbehälter gehalten. Es ist nicht notwendig, dieses bspw. anzuschrauben. Vorzugsweise liegt das Dichtelement somit gegenüberliegend der Stirnkante der Mündungswandung umlaufend (insbesondere vollumfänglich) am Verbindungselement an. Ein Befestigen des Verbindungselements am Milchbehälter führt damit automatisch zu einem Einspannen des Dichtelements.

Es ist bevorzugt, wenn das Verbindungselement einen Verbindungsabschnitt zur Befestigung des Milchbehälters aufweist, und das Dichtelement an dem Verbindungsabschnitt umlaufend anliegt (insbesondere gegenüberliegend und/oder kongruent mit der Stirnkante der Mündungswandung). Vorzugsweise ist der Verbindungsabschnitt zylinderförmig oder konisch ausgebildet.

Es ist vorteilhaft, wenn der Verbindungsabschnitt einen umlaufenden Flansch und an den Flansch anschließend einen Befestigungsfortsatz aufweist, wobei der Befestigungsfortsatz und die Mündungswandung des Milchbehälters zusammenwirkende Befestigungselemente aufweisen, und wobei der Flansch eine (insbesondere umlaufende) Ringnut (insbesondere auf einer der Mündungswandung zugewandten Fläche) aufweist, in die das Dichtelement zumindest teilweise (umlaufend) (insbesondere vollumfänglich) aufgenommen ist. Damit kann das Dichtelement, und damit das Dichtungs- und Ventilelement beim Zusammenbau durch Einlegen in die Ringnut einfach positioniert werden. Weiters wird damit die Dichtwirkung verbessert.

Es ist bevorzugt, wenn das Dichtelement eine umlaufende Auskragung weg von der Mündungswandung aufweist, und die Auskragung (umlaufend) zumindest teilweise in der Ringnut des Flansches aufgenommen ist.

Es ist vorteilhaft, wenn der Verbindungsabschnitt des Verbindungselements und die Mündungswandung des Milchbehälters (insbesondere als zusammenwirkende Befestigungselemente]) jeweils ein Gewinde aufweisen, mit dem der Milchbehälter am Verbindungselement anschraubbar ist, wobei das Dichtelement das Einschrauben des Milchbehälters am Verbindungselement begrenzt. Vorzugsweise ist die Höhe des Dichtelements derart dimensioniert, dass beim Einschrauben die vorgegebene Endposition erreicht wird und in der vorgegebenen Endposition Dichtheit zwischen Milchbehälter und Verbindungselement vorliegt. Vorzugsweise ist das Dichtelement derart ausgelegt, dass es innerhalb eines definierten Bereichs abdichtet.

Es ist vorteilhaft, wenn das Dichtelement einen umlaufenden Dichtflansch aufweist, der (insbesondere vollumfänglich) an einer inneren Mantelfläche der Mündungswandung anliegt. Der Dichtflansch erstreckt sich vorzugsweise weg vom Verbindungselement, in Richtung des Milchbehälters und/oder in eine Richtung, in der das Verbindungselement auf den Milchbehälter geschoben/geschraubt wird. Damit kann die Abdichtung verbessert werden.

Es ist bevorzugt, wenn das Verbindungselement einen Kanal aufweist, der den Hohlraum des Milchbehälters mit dem Nippeltunnel verbindet, wobei der Kanal milchbehälterseitig einen Mündungsabschnitt aufweist, wobei das Ventil- und Dichtungselement, insbesondere das Rückschlagventil, auf den Mündungsabschnitt aufgeschoben ist. Vorzugsweise weist das Ventil- und Dichtungselement, insbesondere das Rückschlagventil, einen (vorzugsweise zylinderförmigen) Halteflansch auf, der auf den Mündungsabschnitt des Kanals aufgeschoben ist, und diese vorzugsweise umfasst. Das Ventil- und Dichtungselement, insbesondere der Halteflansch, hält vorzugsweise reibschlüssig am Mündungsabschnitt. Damit wird die Positionierung bei der Montage vereinfacht. Das Ventil- und Dichtungselement wird bei der Montage einfach am Mündungsabschnitt aufgeschoben. Andererseits wird ein Milchflussweg (vom Kanal zum Rückschlagventil) abgedichtet.

Wenn die Milch in den Milchbehälter gepumpt wird, würde in diesem ein Überdruck entstehen, der das weitere Abpumpen behindert (vgl. Fig. 13 der EP 4066870 A2). Dies ist insbesondere ein Problem bei Brustpumpen, bei denen das Pumpelement keine Luft in die Umgebung abgibt, sondern zyklisch arbeitet bzw. zyklisch Luft ansaugt, beispielsweise eine zyklische Membranpumpe (die den Vorteil hat, dass sie unempfindlich gegen Verunreinigungen durch die Milch und gegen Dauerbeanspruchung ist) und wenn der Milchbehälter starr ausgeführt ist. Daher ist es bekannt, dass der Milchbehälter oder ein Anschlusselement des Verbindungselements mit dem Milchbehälter eine oder mehrere Bohrungen aufweist, durch die Luft nach außen dringen kann und die der Entlüftung des Milchbehälters dient. Solche Anordnungen werden beispielsweise bei Brustpumpen verwendet, die (unter der Kleidung) tragbar sind. Nachteiligerweise kann durch eine solche Entlüftungsbohrung Milch aus dem Milchbehälter austreten. Daher wird die Bohrung üblicherweise möglichst weit oben (in der Betriebsstellung der Brustpumpe beim Abpumpen) vorgesehen, d.h. insbesondere am Anschlusselement des Verbindungselements mit dem Milchbehälter. Wenn die Brustpumpe schief gehalten wird oder sich die abpumpende Person vorbeugt, kann jedoch dennoch Milch austreten und verloren gehen. Es ist daher eine weitere Aufgabe der vorliegenden Erfindung, einen Austritt von Milch aus der Milchpumpe, insbesondere bei einer Schrägstellung der Milchpumpe, durch Entlüftungsöffnungen oder dergleichen zu verringern.

Einerseits wird ein Austritt von Milch durch das Dichtungselement verhindert.

Es ist vorteilhaft, wenn der Verbindungssteg zumindest eine Entlüftungsöffnung aufweist. Durch die Entlüftungsöffnung kann eine der Hohlraum entlüftet und ein Überdruck abgeführt werden. Der Verbindungssteg ist auf der Oberseite des Milchbehälters, insbesondere innerhalb der Mündungswandung, angeordnet. Durch die zentralere Anordnung der Entlüftungsöffnung (gegenüber einer Öffnung in der Wandung/Mantelfläche selbst), wird damit bei einem Schrägstellen bis zu einem steileren Winkelgrad ein Milchaustritt verhindert. Da vorzugsweise der Kanal des Verbindungselements direkt mit Rückschlagventil verbunden ist, ist der Kanal insbesondere von der Entlüftungsöffnung getrennt, bzw. steht mit dieser nur über das Rückschlagventil und den Milchbehälter in fluidischer Verbindung.

Vorzugsweise hat die zumindest eine Entlüftungsöffnung die Form eines Rundlochs oder Langlochs. Vorzugsweise sind 1, 2, 3, 4, 5 oder mehr Entlüftungsöffnungen vorgesehen. Vorzugsweise sind zumindest 2, zumindest 3, zumindest 4 oder zumindest 5 Entlüftungsöffnungen vorgesehen. Vorzugsweise sind zumindest zwei Entlüftungsöffnungen vorgesehen, die um einen Winkel von zumindest 90° (in Bezug auf ein Zentrum des Verbindungsstegs, insbesondere das Rückschlagventil) versetzt zueinander im Verbindungssteg vorgesehen sind.

Das Verbindungselement kann bspw. eine Entlüftungsöffnung zur Umgebung aufweisen, die die Entlüftungsöffnung des Verbindungsstegs mit der Umgebung verbindet.

Es ist bevorzugt, wenn das Verbindungselement eine Verbindungselement-Entlüftungsöffnung aufweist, die in einem Wandabschnitt des Verbindungselements ausgebildet ist, sodass die Verbindungselement-Entlüftungsöffnung über die Entlüftungsöffnung des Verbindungsstegs mit dem Hohlraum des Milchbehälters in (insbesondere fluidischer) Verbindung steht. Vorzugsweise ist der Wandabschnitt dem Verbindungssteg zugewandt und/oder der Wandabschnitt begrenzt zusammen mit dem Ventil- und Dichtungselement einen Raum. Die Verbindungselement-Entlüftungsöffnung führt vorzugsweise nicht zum (Milchfluss- )Kanal. Damit ist der von der Milch zum Austreten zurückzulegende weg noch weiter verlängert und damit der Milchaustritt verringert.

Es ist vorteilhaft, wenn die Milchpumpe einen Entlüftungskanal aufweist, dessen erstes Ende durch die Verbindungselement-Entlüftungsöffnung gebildet wird und dessen zweites Ende offen zur Umgebung ist.

Es ist vorteilhaft, wenn die Verbindungselement-Entlüftungsöffnung auf einer Seite zum Nippeltunnel (insbesondere zu einer äußeren Umfangsfläche des Nippeltunnels) (und auf der anderen Seite zur Entlüftungsöffnung im Verbindungssteg oder zu einem Raum, in den die Entlüftungsöffnung im Verbindungssteg mündet) mündet.

Es ist vorteilhaft, wenn der Entlüftungskanal zumindest abschnittsweise entlang des Nippeltunnels verläuft.

Es ist vorteilhaft, wenn der Nippeltunnel (insbesondere eine äußere Mantelfläche des Nippeltunnels) zumindest eine Nut aufweist, die zumindest einen Abschnitt des Entlüftungskanals bildet. Es ist bevorzugt, wenn der Entlüftungskanal zumindest abschnittsweise gebildet ist durch die Nut im Nippeltunnel (insbesondere einer äußeren Mantelfläche des Nippeltunnels), die in Umfangsrichtung des Nippeltunnels verläuft, wobei vorzugsweise die Verbindungselement-Entlüftungsöffnung zur Nut mündet. Damit kann auf einfache Weise der Entlüftungskanal bereitgestellt und vom Hohlraum weggeführt werden. Es kann auch (alternativ oder zusätzlich) eine (in Umfangsrichtung verlaufende) Nut im Innenumfang (d.h. in der inneren Mantelfläche) des Verbindungselements vorgesehen sein. Vorzugsweise erstreckt sich die in Umfangsrichtung verlaufende Nut im Nippeltunnel vollumfänglich.

Der Entlüftungskanal ist bis auf die beiden Enden vorzugsweise flüssigkeitsdicht. Vorzugsweise verläuft der Entlüftungskanal zumindest abschnittsweise entlang des Verbindungselements. Es ist bevorzugt, wenn der Entlüftungskanal zumindest abschnittsweise entlang eines Abschnitts verläuft, an dem das Verbindungselement den Nippeltunnel aufnimmt und/oder mit dem Nippeltunnel in Kontakt steht. Vorzugsweise mündet Verbindungselement-Entlüftungsöffnung zu dem Bereich zwischen zwei Dichtrippen des Nippeltunnels, die einen Abschnitt des Entlüftungskanals begrenzen können. Es ist vorteilhaft, wenn das Verbindungselement einen Verbindungsabschnitt zur Verbindung mit dem (bzw. zur Aufnahme und/oder zum Einstecken des) Nippeltunnel(s) aufweist, wobei der Verbindungsabschnitt allgemein zylinderförmig oder konisch ausgebildet ist, wobei der Entlüftungskanal zumindest abschnittsweise in Umfangsrichtung des Verbindungsabschnitts des Verbindungselements verläuft. Vorzugsweise verläuft der Entlüftungskanal in Umfangsrichtung über einen Winkelbereich von zumindest 45°, bevorzugt zumindest 90°, noch mehr bevorzugt zumindest 120° oder 150°, des Umfangs des (insbesondere kreiszylinderförmigen) Verbindungsabschnitts.

Es ist bevorzugt, wenn der Entlüftungskanal zumindest abschnittsweise in axialer Richtung des Verbindungsabschnitts des Verbindungselements verläuft. Vorzugsweise schließt der in axiale Richtung verlaufende Abschnitt des Entlüftungskanals an den in Umfangsrichtung verlaufenden Abschnitt des Entlüftungskanals an (oder umgekehrt). Es ist vorteilhaft, wenn der Entlüftungskanal zumindest abschnittsweise gebildet ist durch eine (insbesondere einen Abschnitt der bereits oben erwähnten) Nut im Nippeltunnel (insbesondere an einer äußeren Mantelfläche des Nippeltunnels), die in axialer Richtung des Verbindungsabschnitts des Verbindungselements verläuft. Falls der Nippeltunnel umfänglich verlaufende Dichtrippen aufweist, weisen diese insbesondere ggf. Unterbrechungen für die Nut auf. Es kann auch (alternativ oder zusätzlich) eine (axial verlaufende) Nut im Innenumfang des Verbindungselements vorgesehen sein. Die axial verlaufende Nut ist insbesondere auf der der Verbindungselement-Entlüftungsöffnung gegenüberliegenden Seite des Verbindungsabschnitts vorgesehen. Insbesondere wenn ein Abschnitt des Entlüftungskanals durch einen Bereich zwischen den zwei Dichtrippen des Nippeltunnels gebildet ist, weist vorzugsweise eine der Dichtrippen eine Nut auf, um einen axial verlaufenden Abschnitt des Entlüftungskanals zu bildet.

Vorzugsweise ist Ende des Entlüftungskanals zur Umgebung gebildet durch (zumindest) eine Bohrung im Verbindungsabschnitt des Verbindungselements, wobei die Bohrung vorzugsweise auf der einen Seite zur Nut im Nippeltunnel (und/oder Verbindungselement) und/oder zum Bereich zwischen den Dichtrippen und auf der anderen Seite zur Umgebung mündet. Die Bohrung kann radial und/oder tangential verlaufen. Insbesondere alternativ zur Bohrung ist es vorteilhaft, wenn der Entlüftungskanal zu einer umfänglichen Kante des Verbindungsabschnitts des Verbindungselements verläuft, die mit dem Nippeltunnel in Kontakt steht, wobei die Kante des Verbindungsabschnitts und/oder der Nippeltunnel eine radial verlaufende Nut aufweist, über die der Entlüftungskanal zur Umgebung mündet.

Es ist bevorzugt, wenn das Verbindungselement eine Membran aufweist, über die ein Pumpelement einen Unterdruck im Verbindungselement erzeugen kann. Es ist bevorzugt, wenn die Brustpumpe ein elektrisches Pumpenelement aufweist, das über die Membran einen Unterdruck im Verbindungselement erzeugen kann. Vorzugsweise weist die Brustpumpe einen Energiespeicher auf, der mit dem elektrischen Pumpelement verbunden ist.

Die Erfindung wird im Folgenden anhand eines in den Figuren dargestellten bevorzugten Ausführungsbeispiels näher erläutert, das für die Erfindung jedoch nicht einschränkend ist.
Fig. 1 zeigt schematisch einen Schnitt durch eine bevorzugte Ausführungsform einer erfindungsgemäßen Milchpumpe, ohne Pumpelement, Brusthaube und elektrischer Einheit.
Fig. 2 zeigt schematisch die Milchpumpe der Fig. 1 in einer perspektivischen Ansicht (ebenfalls ohne Pumpelement, Brusthaube und elektrischer Einheit).
Fig. 3 zeigt schematisch die (vollständige) Milchpumpe in der Ausführungsform der Fig. 1 von vorne.
Fig. 4 zeigt schematisch die Milchpumpe 1 in der Ausführungsform der Fig. 1 in einer Schnittdarstellung entlang der Ebene A-A in Fig. 3.
Fig. 5 zeigt schematisch den Ausschnitt C in der Fig. 4 in mehr Detail.
Fig. 6a zeigt schematisch ein Ventil- und Dichtungselement der Milchpumpe der Fig. 1, in einer perspektivischen Ansicht.
Fig. 6b zeigt schematisch das Ventil- und Dichtungselement der Milchpumpe der Fig. 1, in einer Draufsicht.
Fig. 7 zeigt schematisch das Verbindungselement der Milchpumpe der Fig. 1, in einer perspektivischen Ansicht.
Fig. 8 zeigt schematisch den Milchbehälter der Milchpumpe der Fig. 1, in einer perspektivischen Ansicht.
Fig. 9a-9d zeigen schematisch alternative Ausführungsformen des Ventil- und Dichtungselements in einer Draufsicht.

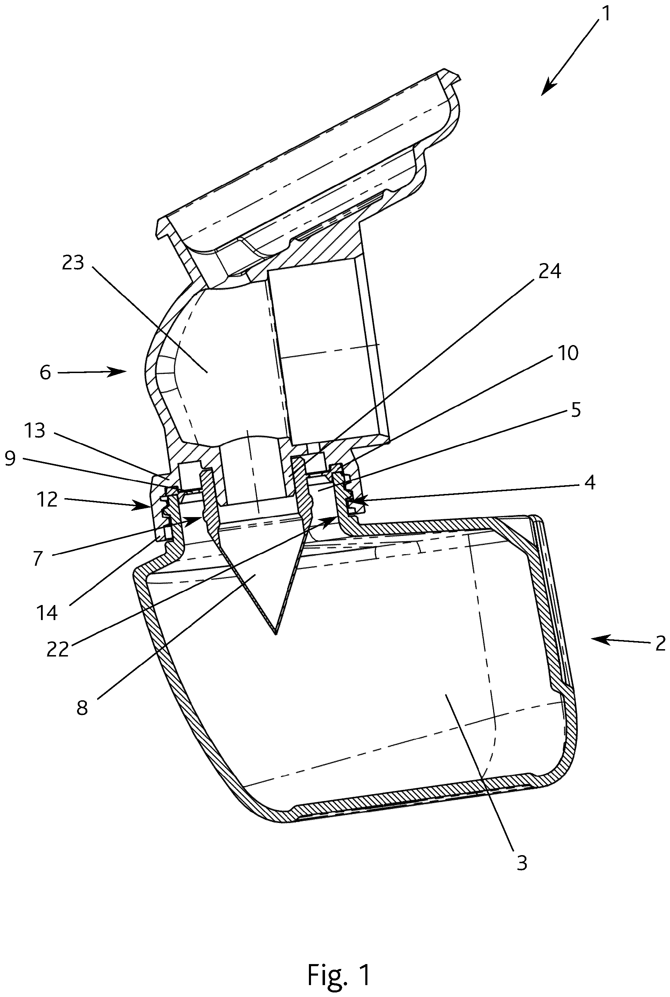

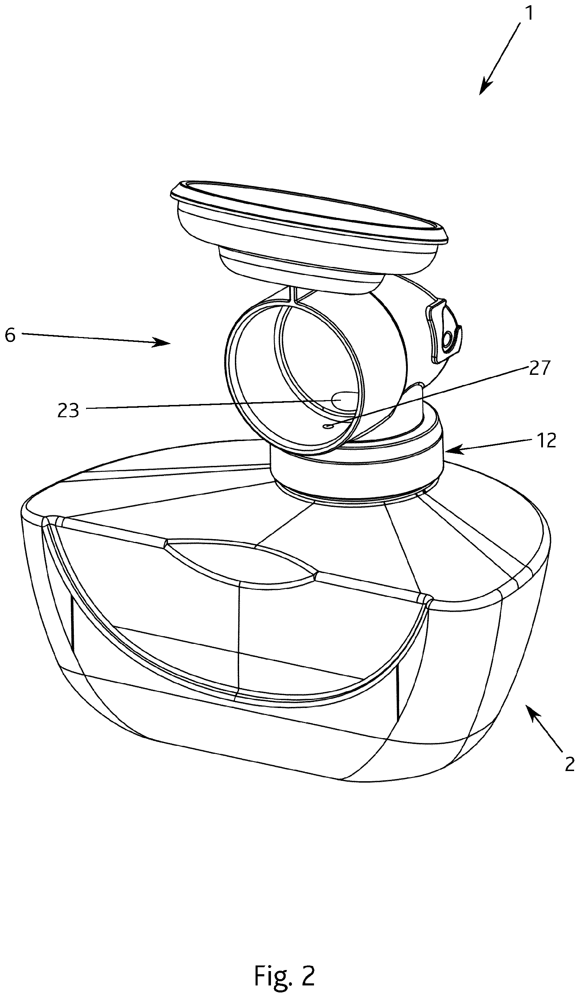

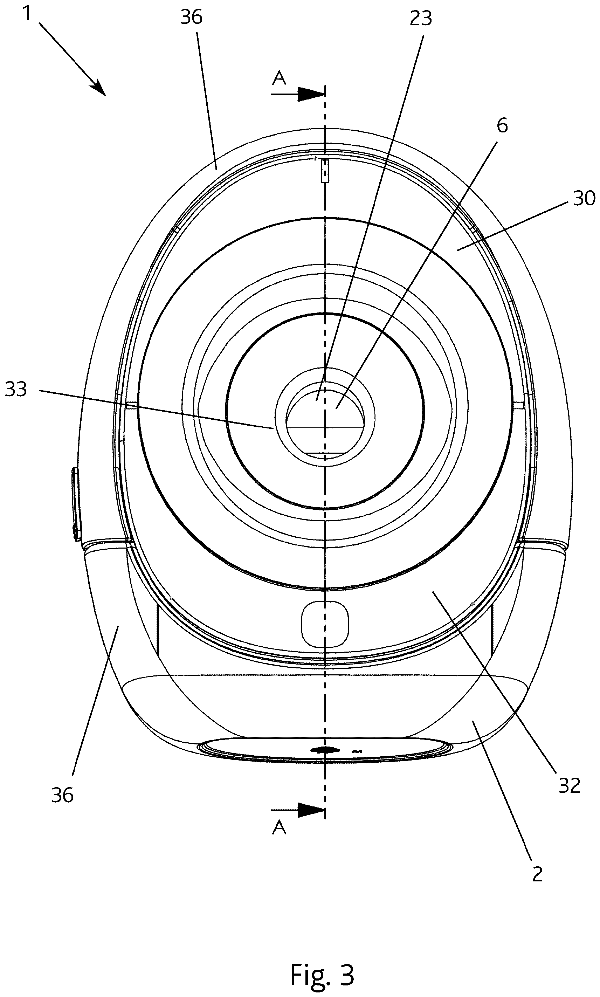

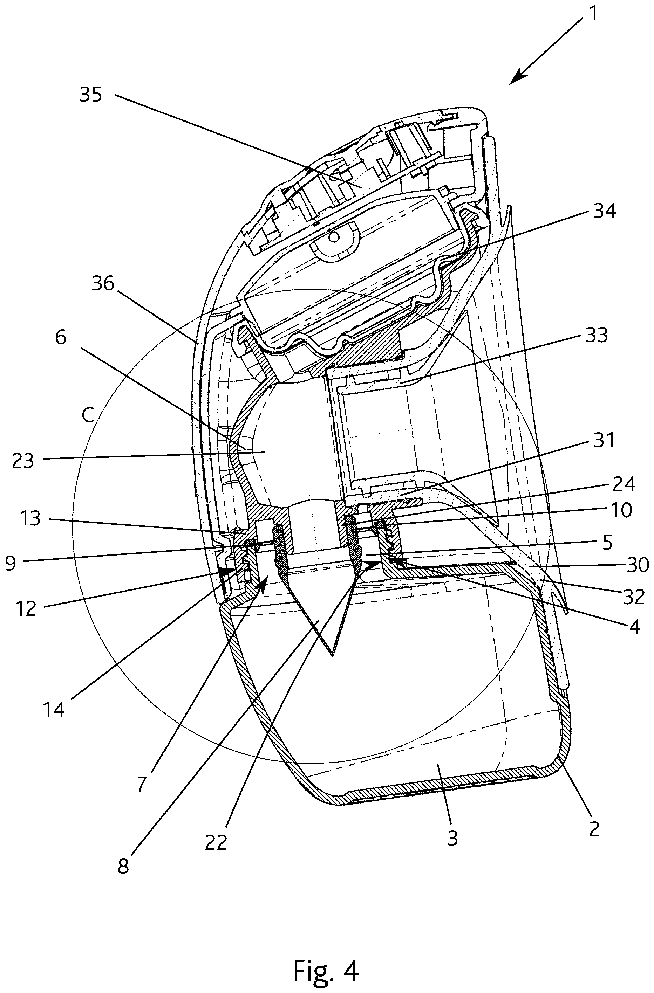

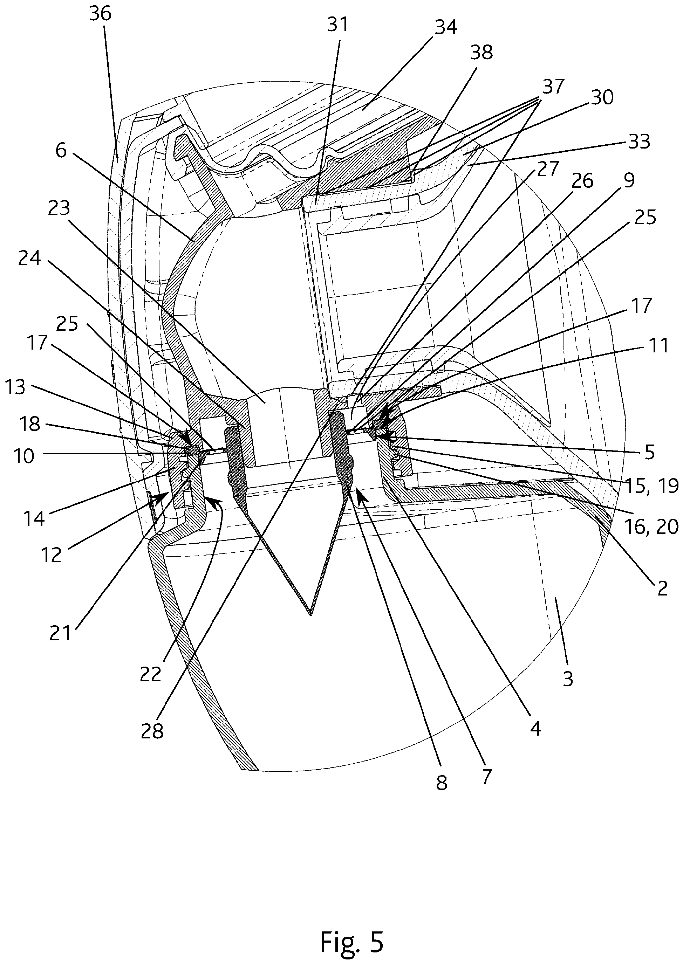

Fig. 1 zeigt schematisch einen Schnitt durch eine bevorzugte Ausführungsform einer erfindungsgemäßen Milchpumpe 1 (ohne Pumpelement, Brusthaube und elektrischer Einheit).. Fig. 2 zeigt die Milchpumpe 1 in einer perspektivischen Ansicht (ebenfalls ohne Pumpelement, Brusthaube und elektrischer Einheit). Fig. 3 zeigt die (vollständige) Milchpumpe 1 von vorne. Fig. 4 zeigt die (vollständige) Milchpumpe 1 in einer Schnittdarstellung entlang der Ebene A-A. (Fig. 1 zeigt einen Schnitt entlang derselben Ebene). Fig. 5 zeigt schematisch den Ausschnitt C in der Fig. 4 größer.

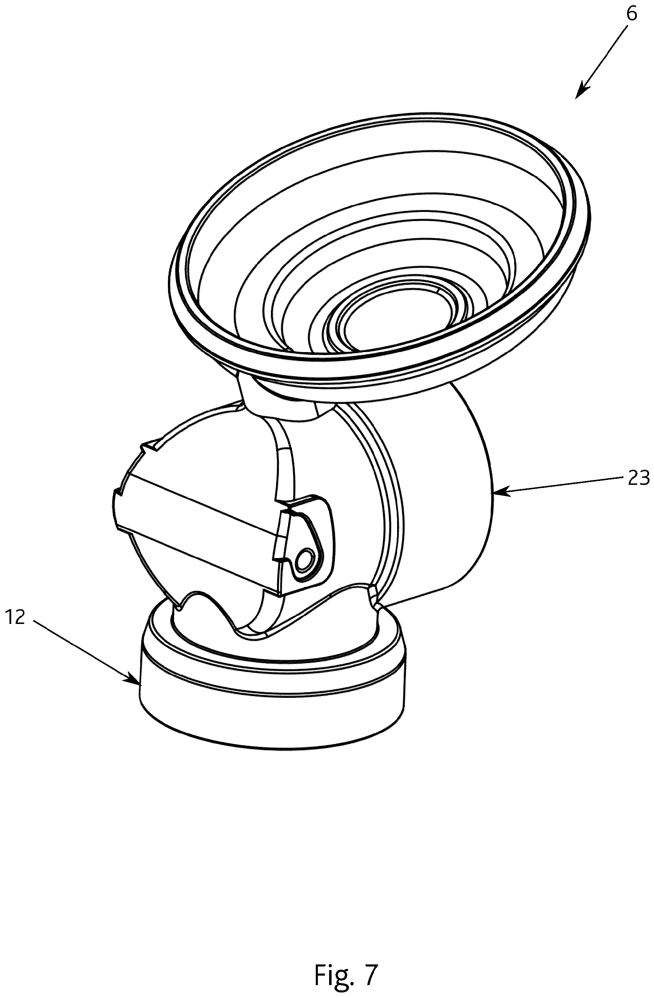

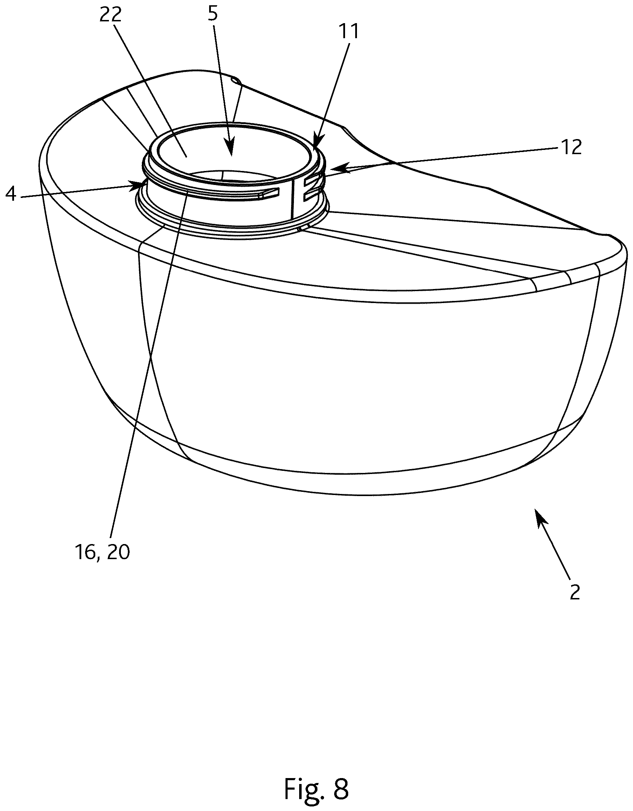

Die Milchpumpe 1 weist einen Milchbehälter 2 mit einem Hohlraum 3 zur Aufnahme von abgepumpter Milch und mit einer umlaufenden Mündungswandung 4, der eine Öffnung 5 zum Hohlraum 3 bildet, eine Brusthaube 30, die einen Brusttrichter 32 und einen Nippeltunnel 31 aufweist, und ein Verbindungselement 6, das die Öffnung 5 zum Hohlraum 3 des Milchbehälters 2 mit dem Nippeltunnel 31 verbindet, auf. In der Brusthaube 30 ist ein Einsatz 33 zur besseren Anpassung an die benutzende Person eingesetzt. Die Milchpumpe 1 weist auch ein Pumpelement 34 (insbesondere eine Membran), eine elektrische Einheit 35 und ein Gehäuse 36 auf. Fig. 7 zeigt schematisch das Verbindungselement 6 in einer perspektivischen Ansicht. Fig. 8 zeigt schematisch den Milchbehälter 2 in einer perspektivischen Ansicht.

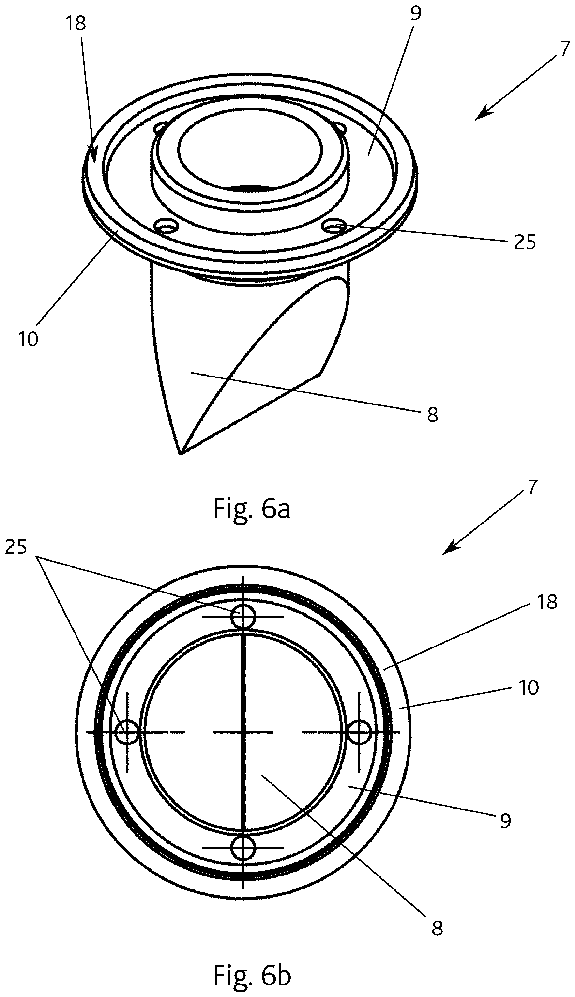

Die Milchpumpe 1 weist weiters ein einstückiges Ventil- und Dichtungselement 7, das ein Rückschlagventil 8, einen sich vom Rückschlagventil 8 erstreckenden umlaufenden Verbindungssteg 9 und ein an den Verbindungssteg 9 anschließendes umlaufendes Dichtelement 10 aufweist. Das Dichtelement 10 liegt vollumfänglich an der Mündungswandung 4 an und dichtet somit zur Mündungswandung 4 hin ab, wodurch ein Milchfluss in den Gewindebereich (s.u.) verhindert wird. Fig. 6a zeigt schematisch das Ventil- und Dichtungselement 7 in einer perspektivischen Ansicht; Fig. 6b zeigt schematisch das Ventil- und Dichtungselement 7 in einer Draufsicht.

Das Dichtelement 10 liegt vollumfänglich an einer Stirnkante 11 (vgl. Fig. 5) der Mündungswandung 4 an. Außerdem liegt das Dichtelement 10 umlaufend um die Öffnung 5 an dem Verbindungselement 6 an, insbesondere gegenüberliegend der Stirnkante 11 des Milchbehälters 2. Das Dichtelement 10 ist somit zwischen dem Verbindungselement 6 und der Mündungswandung 4 des Milchbehälters 2 eingespannt.

Das Verbindungselement 6 weist einen Verbindungsabschnitt 12 zur Befestigung des Milchbehälters 2 auf, wobei das Dichtelement 10 an dem Verbindungsabschnitt 12 anliegt. Im Konkreten weist der Verbindungsabschnitt 12 einen umlaufenden Flansch 13 und an den Flansch 13 anschließend einen Befestigungsfortsatz 14 auf. Der Befestigungsfortsatz 14 weist ein Befestigungselement 15 und die Mündungswandung 4 des Milchbehälters 2 ein Befestigungselement 16 auf, wobei das Befestigungselement 15 des Verbindungselements 6 und das Befestigungselement 16 des Milchbehälters 2 zusammenwirken, wodurch der Milchbehälter 2 am Verbindungselement 6 (lösbar) befestigbar ist. Das Dichtelement liegt am Flansch 13 vollumfänglich an. Im Konkreten weist der Flansch 13 eine Ringnut 17 auf und das Dichtelement 10 weist eine umlaufende Auskragung 18 in Richtung weg von der Mündungswandung 4 auf, wobei die Auskragung 18 in der Ringnut 17 des Flansches 13 aufgenommen ist. Zum Zusammenbauen der Einzelteile der Milchpumpe 1 kann somit einfach das Ventil- und Dichtungselement 7 mit der Auskragung 18 des Dichtelements 10 in die Ringnut 17 des Flansches 13 des Verbindungselements 6 eingelegt werden. Der Zusammenbau ist auch durch die Einstückigkeit des Ventil- und Dichtungselements 7 vereinfacht. Gegenüber einem einfachen Dichtring hat das Dichtelement 7, durch den anschließenden Verbindungssteg 9 eine höhere Stabilität, wodurch das Einlegen in der Ringnut 17 deutlich vereinfacht ist.

Als Befestigungselement 15 des Verbindungselements 6 ist ein Gewinde 19 und als Befestigungselement 16 des Milchbehälters 2 ein Gewinde 20 vorgesehen. Mit den zusammenwirkenden Gewinden 15, 16 ist der Milchbehälter 2 am Verbindungselement 6 anschraubbar. In dieser Ausführungsform ist die Milchpumpe 1 insbesondere als tragbare Version ausgeführt, wobei der Milchbehälter 2 zur Verbesserung der Ergonomie asymmetrisch (also nicht rotationsymmetrisch) ausgeführt ist. Es ist daher vorteilhaft, wenn das Einschrauben des Milchbehälters 2 am Verbindungselement 6 begrenzt wird, damit die Ausrichtung des Milchbehälters 2 relativ zum Verbindungselement 6 vorgegeben ist. Dies wird bei der vorliegenden Milchpumpe 1 erreicht, indem das Dichtelement 10 zwischen Milchbehälter 2 und Verbindungselement 6 vorgesehen ist und das Dichtelement 10 in Einschraubrichtung zwischen dem Milchbehälter 2 und dem Verbindungselement 6 (insbesondere zwischen der Stirnkante 11 der Mündungswandung 4 und dem Flansch 13 des Verbindungsabschnitts 12 des Verbindungselements 6) eingespannt wird. Somit kann über die Höhe des Dichtelements 10 das Einschrauben begrenzt und damit die Ausrichtung des Milchbehälters 2 relativ zum Verbindungselement 6 vorgegeben werden.

Wie ersichtlich, wird ein Austreten von Milch zu den Gewinden 16, 20 und von dort nach außen durch die vollumfängliche Abdichtung des Dichtelements 10 gegenüber der Mündungswandung 4 und gegenüber dem Flansch 13 verhindert.

Um die Abdichtung weiter zu verbessern, weist das Dichtelement 10 weiters einen umlaufenden Dichtflansch 21 auf, der an einer inneren Mantelfläche 22 der Mündungswandung 4 (vollumfänglich) anliegt. Der Dichtflansch 21 erstreckt sich somit vom Dichtelement 10 (oder vom Verbindungssteg 9) in Richtung weg vom Verbindungselement 6 (nach unten).

Das Verbindungselement 6 bildet einen Kanal 23, der den Hohlraum 3 des Milchbehälters 2 mit dem Nippeltunnel 31 verbindet und den Milchflussweg der abgepumpten Milch in den Milchbehälter 2 darstellt. Der Kanal 23 weist milchbehälterseitig einen Mündungsabschnitt 24, wobei das Ventil- und Dichtungselement 7, insbesondere das Rückschlagventil 8, auf den Mündungsabschnitt 24 aufgeschoben ist. Damit wird einerseits eine Abdichtung des Kanals 23 erreicht. Andererseits wird die Ausrichtung des Ventil- und Dichtungselements 7 vereinfacht, da nur das Ventil- und Dichtungselement 7 auf den Mündungsabschnitt 24 aufgeschoben werden muss. Damit ist automatisch auch das Dichtelement 10 richtig in der Ringnut 17 angeordnet.

Zur Entlüftung des Milchbehälters 2 weist der Verbindungssteg 9 zumindest eine Entlüftungsöffnung 25 (in dieser Ausführungsform vier Entlüftungsöffnung 25, siehe Fig. 6b) in Form einer Bohrung auf. Beim Pumpen von Milch in den Milchbehälter entstehender Überdruck kann somit über die Entlüftungsöffnungen 25 abgeführt werden. Die Entlüftungsöffnungen 25 führen insbesondere zu einem Raum 27, der von dem Ventil- und Dichtungselement 7 und dem Verbindungselement 6 begrenzt wird, und der vom Kanal 23 abgegrenzt ist. Um den Überdruck an die Umgebung abzuführen, könnte bspw. das Verbindungselement 6 eine Bohrung vom Raum 27 direkt zur Umgebung aufweisen. In dieser Ausführungsform ist es jedoch vorgesehen, dass das Verbindungselement 6 eine Verbindungselement-Entlüftungsöffnung 26 aufweist, die in einem Wandabschnitt 28 des Verbindungselements 6 ausgebildet ist, sodass die Verbindungselement-Entlüftungsöffnung 26 über die Entlüftungsöffnung 25 des Verbindungsstegs 9 mit dem Hohlraum 3 des Milchbehälters 2 in Verbindung steht. D.h., Luft kann aus dem Milchbehälter 2 über die Entlüftungsöffnung 25 (, den Raum 27,) und die Verbindungselement-Entlüftungsöffnung 26 abgeführt werden.

Die Entlüftungsöffnung 25 könnte auch direkt an der Verbindungselement-Entlüftungsöffnung 26 anliegen (wobei die Entlüftungsöffnung 25 beim Zusammenbauen der Milchpumpe 1 jedoch an der Verbindungselement-Entlüftungsöffnung 26 ausgerichtet werden müsste).

Um den Weg, den Milch beim ungewünschten Austritt über den Entlüftungsweg nehmen muss, weiter zu verlängern und damit den ungewünschten Milchaustritt auf diesem Weg weiter zu verringern, ist ein Entlüftungskanal 37 ausgebildet. Das erste Ende des Entlüftungskanals 37 ist durch die Verbindungselement-Entlüftungsöffnung 26 gebildet und das zweite Ende 38 ist offen zur Umgebung. Dafür mündet die Verbindungselement-Entlüftungsöffnung 26 auf einer Seite zum Nippeltunnel 31. Der Entlüftungskanal 37 verläuft dann in Umfangsrichtung um den Nippeltunnel 31. Dafür kann der Nippeltunnel 31 eine erste Nut, die Umfangsrichtung verläuft, aufweisen. An die erste Nut anschließend weist der Nippeltunnel 31 eine in axialer Richtung verlaufende, zweite Nut auf, sodass der Entlüftungskanal 37 anschließend an die erste Nut in axialer Richtung entlang des Nippeltunnels 31 verläuft. Die zweite Nut verläuft zu einer umfänglichen Kante des Verbindungselements 6, die mit der Brusthaube 30 in Kontakt steht. Der Nippeltunnel 31 weist dort eine dritte Nut auf, die in radialer Richtung verläuft und über die der Entlüftungskanal 37 am zweiten Ende 38 zur Umgebung mündet.

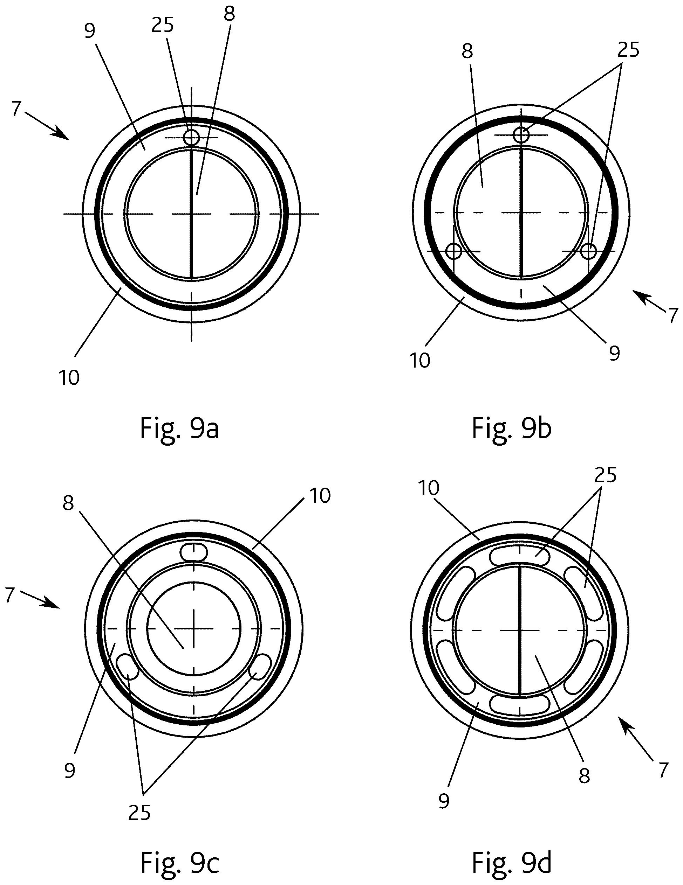

Die Fig. 9a-9d zeigen schematisch alternative Ausführungsformen des Ventil- und Dichtungselements 1 in einer Draufsicht. In Fig. 9a weist der Verbindungssteg 9 eine Entlüftungsöffnung 25 auf, in den Fig 9b und 9c weist der Verbindungssteg 9 drei Entlüftungsöffnungen 25 auf und in Fig. 9d weist der Verbindungssteg sechs Entlüftungsöffnungen 25 auf. In den Fig. 9a und 9b sind die Entlüftungsöffnungen 25 wie in Fig. 6b als Rundlöcher ausgeführt. In den Fig. 9c und 9d sind die Entlüftungsöffnungen 25 als Langlöcher ausgeführt. In Fig. 9d decken die Entlüftungsöffnungen 25 insbesondere einen Großteil des Winkelbereichs des Verbindungsstegs 9 ab.

## Patentansprüche

1. Milchpumpe (1) aufweisend:
- einen Milchbehälter (2) mit einem Hohlraum (3) zur Aufnahme von abgepumpter Milch und mit einer umlaufenden Mündungswandung (4), der eine Öffnung (5) zum Hohlraum (3) bildet;
- eine Brusthaube (30) mit einem Nippeltunnel (31);
- ein Verbindungselement (6), das die Öffnung (5) zum Hohlraum (3) des Milchbehälters (2) mit dem Nippeltunnel (31) verbindet;
**gekennzeichnet durch**
ein einstückiges Ventil- und Dichtungselement (7), das ein Rückschlagventil (8), einen sich vom Rückschlagventil (8) erstreckenden umlaufenden Verbindungssteg (9) und ein an den Verbindungssteg (9) anschließendes umlaufendes Dichtelement (10) aufweist,
wobei das Dichtelement (10) vollumfänglich an der Mündungswandung (4) anliegt.

2. Milchpumpe (1) nach Anspruch 1, wobei das Dichtelement (10) vollumfänglich an einer Stirnkante (11) der Mündungswandung (4) anliegt.

3. Milchpumpe (1) nach einem der vorhergehenden Ansprüche, wobei das Dichtelement (10) umlaufend um die Öffnung (5) an dem Verbindungselement (6) anliegt.

4. Milchpumpe (1) nach einem der vorhergehenden Ansprüche, wobei das Verbindungselement (6) einen Verbindungsabschnitt (12) zur Befestigung des Milchbehälters (2) aufweist, und das Dichtelement (10) an dem Verbindungsabschnitt (12) anliegt.

5. Milchpumpe (1) nach Anspruch 4, wobei der Verbindungsabschnitt (12) einen umlaufenden Flansch (13) und an den Flansch (13) anschließend einen Befestigungsfortsatz (14) aufweist, wobei der Befestigungsfortsatz (14) und die Mündungswandung (4) des Milchbehälters (2) zusammenwirkende Befestigungselemente (15, 16) aufweisen, und wobei der Flansch (13) eine Ringnut (17) aufweist, in die das Dichtelement (10) zumindest teilweise aufgenommen ist.

6. Milchpumpe (1) nach Anspruch 5, wobei das Dichtelement (10) eine umlaufende Auskragung (18) in Richtung weg von der Mündungswandung (4) aufweist, und die Auskragung (18) zumindest teilweise in der Ringnut (17) des Flansches (13) aufgenommen ist.

7. Milchpumpe (1) nach einem der Ansprüche 4 bis 6, wobei der Verbindungsabschnitt (12) des Verbindungselements (6) und die Mündungswandung (4) des Milchbehälters (2) jeweils ein Gewinde (19, 20) aufweisen, mit dem der Milchbehälter (2) am Verbindungselement (6) anschraubbar ist, wobei das Dichtelement (10) das Einschrauben des Milchbehälters (2) am Verbindungselement (6) begrenzt.

8. Milchpumpe (1) nach einem der vorhergehenden Ansprüche, wobei das Dichtelement (10) einen umlaufenden Dichtflansch (21) aufweist, der an einer inneren Mantelfläche (22) der Mündungswandung (4) anliegt.

9. Milchpumpe (1) nach einem der vorhergehenden Ansprüche, wobei das Verbindungselement (6) einen Kanal (23) aufweist, der den Hohlraum (3) des Milchbehälters (2) mit dem Nippeltunnel (31) verbindet, wobei der Kanal (23) milchbehälterseitig einen Mündungsabschnitt (24) aufweist, wobei das Ventil- und Dichtungselement (7), insbesondere das Rückschlagventil (8), auf den Mündungsabschnitt (24) aufgeschoben ist.

10. Milchpumpe (1) nach einem der vorhergehenden Ansprüche, wobei der Verbindungssteg (9) zumindest eine Entlüftungsöffnung (25) aufweist.

11. Milchpumpe (1) nach Anspruch 10, wobei das Verbindungselement (6) eine Verbindungselement-Entlüftungsöffnung (26) aufweist, die in einem Wandabschnitt des Verbindungselements (6) ausgebildet ist, sodass die Verbindungselement-Entlüftungsöffnung (26) über die Entlüftungsöffnung (25) des Verbindungsstegs (9) mit dem Hohlraum (3) des Milchbehälters (2) in Verbindung steht.

12. Milchpumpe (1) nach Anspruch 11, aufweisend einen Entlüftungskanal (37), dessen erstes Ende durch die Verbindungselement-Entlüftungsöffnung (26) gebildet wird und dessen zweites Ende (38) offen zur Umgebung ist.

13. Milchpumpe (1) nach Anspruch 11 oder 12, wobei die Verbindungselement-Entlüftungsöffnung (26) auf einer Seite zum Nippeltunnel (31) mündet.

14. Milchpumpe (1) nach Anspruch 12 und 13, wobei der Entlüftungskanal (37) zumindest abschnittsweise entlang des Nippeltunnels (31) verläuft.

15. Milchpumpe (1) nach Anspruch 14, wobei der Nippeltunnel (31) zumindest eine Nut aufweist, die zumindest einen Abschnitt des Entlüftungskanals (37) bildet.
